# EUROPEAN PATENT SPECIFICATION

(11) **EP 3 669 983 B1**
(45) Date of publication and mention of the grant of the patent: **01.06.2022**
(21) Application number: 19214362.6
(22) Date of filing: 09.12.2019
(51) Int. Cl.: B01L 3/00, C12M 1/34, G01N 33/52, G01N 33/53

(54) **A LID OF A HOLDER OF A MEMBRANE FOR THE TRANSFER OF BIOMOLECULES AND THE HOLDER OF THE MEMBRANE FOR THE TRANSFER OF BIOMOLECULES FOR PERFORMING A TECHNIQUE OF DOT BLOT**
DECKEL EINES HALTERS EINER MEMBRAN ZUM TRANSFER VON BIOMOLEKÜLEN UND HALTER DER MEMBRAN ZUM TRANSFER VON BIOMOLEKÜLEN ZUR DURCHFÜHRUNG EINER TECHNIK EINES DOTBLOTS
COUVERCLE D'UN SUPPORT D'UNE MEMBRANE POUR LE TRANSFERT DE BIOMOLÉCULES ET SUPPORT DE MEMBRANE POUR LE TRANSFERT DE BIOMOLÉCULES POUR EFFECTUER UNE TECHNIQUE DE BUVARDAGE EN TACHES

(30) Priority: 17.12.2018 CZ 20180706
(43) Date of publication of application: 24.06.2020
(73) Proprietor: Univerzita Pardubice, 530 09 Pardubice, Polabiny (CZ); Ustav Analyticke Chemie AV CR, V.V.I., 60200 Brno (CZ)
(72) Inventor: Svobodová, Zuzana, 530 02 Pardubice (CZ); Novotný, Jakub, 735 14 Orlová, Lutyne (CZ); Foret, Frantisek, 621 00 Brno, Oresín (CZ); Bílková, Zuzana, 533 04 Sezemice (CZ)
(74) Representative: Musil, Dobroslav

(56) References cited:
- EP-A1- 0 301 141
- US-A1- 2004 185 577
- US-A1- 2017 233 790
- Nidhi Vishnoi: "Bio-Dot Microfiltration Apparatus From Bio-Rad", Biocompare.com, 20 February 2008 (2008-02-20), XP055671455, Retrieved from the Internet: URL:https://www.biocompare.com/Product-Rev iews/40418-Bio-Dot-Microfiltration-Apparat us-From-Bio-Rad/ [retrieved on 2020-02-25]

## Description

### Technical field

The present invention relates to a lid of a holder of a membrane for the transfer of biomolecules for performing a technique of dot blot, the lid being provided with through dispensing apertures.

The invention also relates to a holder of the membrane for the transfer of biomolecules for performing the technique of dot blot using this lid.

### Background art

At present, the situation on the market for antibodies used for research and diagnostic purposes is rather confusing - antibodies often exhibit different parameters from those declared by manufacturers. Characteristics, such as specificity, affinity, cross-reactivity, absolute quantity, purity of the formulation, chemical structure of immunoglobulins, including post-translational modifications, are of crucial importance for their final use. Differences in the so-called "reactivity" of antibodies having identical parameters declared by manufacturers are often enormous; differences also occur between batches of one product (this applies especially to polyclonal antibodies), etc. - see, e.g., publications A. Bradbury a. Plückthun: "Reproducibility: Standardize antibodies used in research", Nature Vol. 518, 27-29, 2015 and M. Baker: "Reproducibility crisis: Blame it on the antibodies", Nature, Vol. 521, 274-276, 2015, etc. What is more, there are large price differences, where an antibody of the same declared parameters is offered by different manufacturers at considerably different prices and in different quality. Currently, proven methods, such as enzyme immunoassay methods (more often ELISA - Enzyme-linked Immunosorbent Assay) or "westernblot", are used to verify the declared reactivity of antibodies. However, the above-mentioned methods are relatively laborious, time consuming and require an experienced worker to correctly interpret the results. Another alternative is using instrumental methods, i.e. methods based on Surface Plasmon Resonance (SPR), isothermal titration calorimetry (ITC) or immunoprecipitation combined with mass spectrometry (IP-MS), which, nevertheless, require very high acquisition costs and the obtained data are difficult to interpret. Therefore, not only for these reasons, but also due to the high cost of the antibodies themselves, which would degrade during the verification of their characteristics, a substantial proportion of researchers do not test the antibodies and trust the information provided by the manufacturers. The use of antibodies having other than the declared parameters then often results in misrepresentation or degradation of the whole research and consequently in a considerable waste of time, materials and money. For example, it has been found that in the USA alone, the use of low-quality antibodies in research leads to wasting over 350 million dollars a year (see A. Bradbury and Plückthun: "Reproducibility: Standardized Antibodies Used in Research", Nature Vol. 518, 27-29, 2015).

Dot blot is an analytical method based on transferring biomolecules (proteins/peptides, nucleic acids, etc.) from a mobile phase (e.g. a liquid solution) to a solid phase (a membrane for the transfer of biomolecules - a blotting membrane, e.g. nitrocellulose, polyvinylidene difluoride or nylon) and fixing the biomolecules on the membrane. The solution of the particular biomolecule/biomolecules is then pipetted into separate dispensing apertures formed in a lid of a membrane holder and filtered by means of vacuum through an appropriate membrane for the transfer of biomolecules, on which the biomolecule/biomolecules is/are fixed by physical forces and dots (targets) are formed on it which are able to interact with other reagents for the detection and identification of these biomolecules - e.g., with more easily detectable molecules or with antibodies, etc. For semi-quantitative identification of different biomolecules, the respective membrane for the transfer of biomolecules must be removed from the membrane holder after dot formation and incubated with an appropriate reagent; for semi-quantitative determination of the degree of cross-reactivity of different biomolecules, it is necessary to remove the membrane for the transfer of biomolecules from the holder after dot formation and cut it into parts with individual dots and then incubate each of these parts separately with a respective reagent or a biomolecule, e.g., in a Petri dish, a divided Petri dish, and the like. However, this procedure is manually demanding (involving a series of incubations with different reagents with numerous washing steps) and, moreover, there is a certain risk of confusing dots which contain different biomolecules tested.

US 2017/233790 discloses an array device utilisable in Invader analytic method or ELISA (Enzyme-Linked ImmunoSorbent Assay) method, but not in technique of dot blot. The array device includes a base having plurality of wells, a cover, an injection port, a discharge port and an aqueous solution. The base includes a substrate and a microporous array layer with arrayed through holes. These through holes are from the lower side closed by the base. Between the microporous array layer and the lower surface of the cover, there is a gap of 100 µm serving as channel for transport of various liquids, such as aqueous solution, detection reaction reagent, oleaginous sealing liquid, etc. This gap/channel interconnects the injection port with the discharge port. Also another variants of this array device having different design of the cover are disclosed. For example, variant in which the cover can have more injection ports, whereby to each of the injection ports a discharge port is allocated and possibly also a waste liquid vessel for waste/excess liquid. Nevertheless, the general principle is common for all variants - all injection ports and all discharge ports are interconnected with each other by means of a gap between the microporous array layer and the cover.

The object of the invention is to provide a lid of a holder of a membrane for the transfer of biomolecules for performing a technique of dot blot, which would eliminate the disadvantages of the background art and which would simplify and accelerate semi-quantitative identification of biomolecules and semi-quantitative determination of the degree of their mutual reactivity, thus enabling, among other things, easy, fast and inexpensive verification of the characteristics and parameters of antibodies declared by manufacturers.

In addition, it is an object of the invention to provide a holder of the membrane for the transfer of biomolecules with this lid.

### Principle of the invention

The object of the invention is achieved by a lid of a holder of a membrane for the transfer of biomolecules for performing a technique of dot blot in which through dispensing apertures are formed, whose principle consists in that at least one discharge aperture is formed in the lid, whereby the through dispensing apertures and the discharge aperture are interconnected in the vicinity of the orifice of the through dispensing apertures on the underside of the lid via discharge channels which allow to discharge washing solutions and reagents or other materials from the through apertures. Each discharge channel has a cross-sectional area having a size of a maximum of 12000 µm², preferably a maximum of 10000 µm², wherein there is no spontaneous drainage of liquid from the through dispensing aperture and is provided with at least one sealing chamber and/or with a hydrophobic coating or finish on at least a portion of its length to prevent undesired backflow into the respective through dispensing aperture.

The discharge channels and discharge aperture/apertures are interconnected directly or via collecting channels with a larger cross-sectional area.

For proper alignment with the other parts of the membrane holder, the lid according to the invention is on its underside provided with a recess, e.g., of a square shape, whereby the dispensing apertures open into this recess.

To determine the degree of mutual reactivity of two complementary biomolecules, it is, for example, advantageous if the through dispensing apertures in the lid of the holder of the membrane for the transfer of biomolecules are arranged in a square matrix, which during a chaotropic reagent step of allows the lid to rotate in a horizontal plane by 90 ° while maintaining the alignment of the biomolecule-containing dots on the membrane for the transfer of biomolecules and the through dispensing apertures for dispensing a chaotropic reagent.

The discharge channels preferably have a rectangular cross-section with a width of 200 µm and a depth of 50 µm.

In an embodiment of the simplest construction, the discharge channels are configured as open channels on the underside of the lid of the holder of the membrane for the transfer of biomolecules.

Preferably, the discharge aperture/apertures is/are provided with a quick coupling for easy and quick connection and disconnection of a vacuum source.

In a preferred variant of embodiment, the discharge aperture/apertures is/are formed in at least one lateral side of the lid.

To simplify the process of dispensing solutions and reagents, it is advantageous if a dispensing container for each through dispensing aperture is formed in the upper side of the lid. Such a dispensing container may be formed for each dispensing aperture separately, e.g. by expanding the inlet to the dispensing aperture, and/or at least some dispensing containers may be common to a group of adjacent dispensing apertures.

In addition to the lid of the holder of the membrane for the transfer of biomolecules, the aim of the invention is achieved by a holder of the membrane for the transfer of biomolecules for performing the technique of dot blot, which comprises a lid with through dispensing apertures, a base with through apertures which are in alignment with the through dispensing apertures of the lid and a bottom with a recess arranged under the through apertures of the base, the recess being connected to a discharge aperture, whose principle consists in that the lid of the holder of the membrane for the transfer of biomolecules is formed by the lid according to the invention.

If the discharge channels and/or the collecting channels and/or the sealing chambers of the discharge channels are configured as open channels on the underside of the lid, it is necessary for the membrane for the transfer of biomolecules to be arranged between two layers of foil which is provided with apertures which are in alignment with the dispensing apertures of the lid.

### Description of drawings

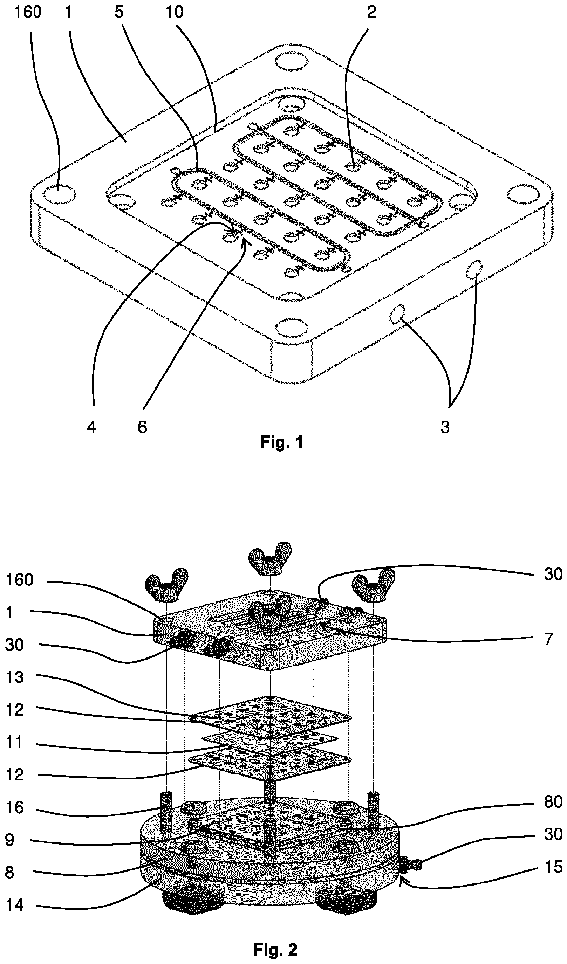

In the enclosed drawing, Fig. 1 schematically represents a lid of a holder of a membrane for the transfer of biomolecules for performing the technique of dot blot according to the invention in a preferred embodiment, seen from below, and Fig. 2 shows a partially exploded view of the holder of the membrane for the transfer of biomolecules for performing the technique of dot blot according to the invention, including the membrane for the transfer of biomolecules, which comprises the lid in the embodiment shown in Fig. 1.

### Examples of embodiment

Known through dispensing apertures **2** are formed in a lid **1** of a holder of a membrane for the transfer of biomolecules for performing the technique of dot blot according to the invention. In the variant of embodiment shown, the through dispensing apertures **2** have a circular cross-section, but not necessarily so. The dispensing apertures **2** are preferably arranged in a square matrix at a spacing corresponding in both directions to the spacing of the channels of a standard multichannel pipette. In addition, at least one discharge aperture **3** is formed in the lid **1**, whereby the through dispensing apertures **2** and the discharge aperture/apertures **3** are interconnected via discharge channels **4** formed in the vicinity of the orifice of the dispensing apertures **2** on the underside of the lid **1,** or at least some of them are interconnected directly. The discharge aperture/apertures **3** is/are preferably provided with a quick coupling **30** (Fig. 2) for easy connection and disconnection of an unillustrated vacuum source. In the embodiment shown in Figs. 1 and 2, in total four discharge apertures **3** are formed in two lateral walls of the lid **1,** arranged in pairs opposite each other, whereby the discharge channels **4** are made as open channels on the lower surface of a square recess **10** which is formed on the underside of the lid **1** and are connected to collecting channels **5** which are configured in the same manner but have a larger cross-sectional area and are connected to individual discharge apertures **3.** In a variant of embodiment (not shown), both the discharge channels **4** and the collecting channels **5** may be configured as closed channels in the body of the lid **1.** These discharge channels **4** allow the liquid (especially washing solutions, reagents, etc.) to be pumped out of the individual dispensing apertures **2** by means of a vacuum source (not shown) which is connected, with an interposed safety vessel, to the discharge aperture/apertures **3.** Due to this, as will be described in more detail below, it is not necessary to remove the membrane for the transfer of biomolecules from the holder during the determination of the degree of mutual reactivity of a target and complementary biomolecule and verification of a possibility of binding a complementary biomolecule to a non-target biomolecule, because the required solutions and reagents can be applied directly to the membrane for the transfer of biomolecules, or to biomolecule-containing dots formed thereon through the dispensing apertures **2** of the lid **1**, and the remainder or excess of the biomolecules is then removed from the surface of the membrane for the transfer of biomolecules by means of the discharge channels **4.** Since during using this lid **1,** the biomolecule-containing dots are washed as the washing solution flows through the dispensing apertures **2** into the discharge channels 4, i.e. non-statically, a higher washing efficiency is achieved, which allows, for example, to decrease incubation time and/or reduce the number of washing steps.

In addition, the variant of the lid **1** according to the invention in which the dispensing apertures **2** are arranged in a square matrix, allows to include a chaotropic step in the analysis, wherein a chaotropic reagent is applied at different concentrations to the membrane for the transfer of biomolecules perpendicular to the direction of the other reagents and solutions to be applied, without the need to remove the membrane for the transfer of biomolecules from the holder - in doing this, the lid **1** of the holder is only lifted and rotated by 90 ° in a horizontal plane and then placed back on the other parts of the holder with the membrane for the transfer of biomolecules, whereby the dispensing apertures **2** of the holder are aligned with the dots containing a biomolecule/biomolecules.

Each discharge channel **4** has a cross-sectional area of a maximum of 12000 µm², preferably a maximum of 10000 µm², without spontaneous leakage of liquid from the respective through dispensing aperture **2.** In a preferred variant of embodiment, the discharge channels **4** have a rectangular cross-section with a width of 200 µm and a depth of 50 µm. Prior to being connected to the collecting channel **5** or into the discharge aperture **3,** each discharge channel **4** is provided with a means to prevent spontaneous leakage of liquid (e.g., of the washing solution or reagent, etc.) from the dispensing aperture **2** in the lid **1** through this channel and at the same time to prevent the risk of backflow to the dispensing aperture **2,** whereby the liquid contained therein could be contaminated with liquid from another dispensing aperture/other dispensing apertures **2** (for example, of two antibodies to be compared or a chaotropic reagent at different concentrations). Such a means is, for example, at least one sealing chamber **6** formed by an enlarged channel **4** and/or a surface finish or layer on at least a portion of the length of the channel **4** - e.g., a hydrophobic layer (e.g. based on titanium oxide/oxides or a commercial silicone based hydrophobic coating) or hydrophobic chemical modification of walls (e.g. based on silanization or introduction of fluorosilanes, etc.). In a preferred variant of embodiment of the sealing chambers **6,** the walls of the chambers **6** are connected to the walls of the discharge channels **4** at right angles, when the efficiency of the sealing chambers **6** is the highest.

In the upper side of the lid **1** are preferably formed dispensing containers **7**, which are aligned with the dispensing apertures **2** and which serve to facilitate dispensing the solution of the biomolecule/biomolecules or washing solutions and reagents into the dispensing apertures **2.** The dispensing containers **7** may be formed separately for each dispensing aperture **2,** for example, by enlarging the inlet to the dispensing aperture **2** and/or may be common to a group of adjacent dispensing apertures **2** - see, e.g., the embodiment shown in Fig. 2, in which one common dispensing container **7** is formed for each row of dispensing apertures **2.** The dispensing containers **7** then facilitate dispensing the solution of the biomolecule/biomolecules, of the washing solutions and reagents into the individual dispensing apertures **2.**

The holder of the membrane for the transfer of biomolecules for performing the technique of dot blot according to the invention comprises in addition to the lid **1** a well-known **base 8** which is on its upper side provided with an elevation **80,** corresponding to the recess **10** on the underside of the lid **1.** Formed in the base **8** are through apertures **9** which are aligned with the dispensing apertures **2** of the lid **1**. Preferably, the through apertures **9** are getting enlarged towards the underside of the base **8,** which facilitates removal of the liquid filtered through the membrane for the transfer of biomolecules and reduces the risk of their accumulation in the space below the membrane. Preferably, the **base 8** is provided on its upper side with means (not shown) for fixing the membrane for the transfer of biomolecules temporarily and preventing it from moving when the lid **1** is being removed and possibly rotated. On the elevation **80** and/or around the elevation **80,** the base **8** is provided with a sealing layer (not shown), preferably of microporous rubber, which serves to mutually seal the base **8** and the lid **1.** If this sealing layer is arranged directly on the surface of the elevation **80** of the base **8,** it is provided with apertures which are aligned with the dispensing apertures **2** of the lid **1.**

A known membrane **11** for the transfer of biomolecules (a so-called blotting membrane, e.g., nitrocellulose, polyvinylidene difluoride, nylon, etc.) is then inserted between the lid **1** and the base **8** of the holder of the membrane for the transfer of biomolecules. If the discharge channels **4** and/or the collecting channels **5** and/or the sealing chambers **6** of the discharge channels **4** are formed on the underside of the lid **1** as open, it is necessary for the membrane **11** for the transfer of biomolecules to be arranged between two layers of foil **12** which is provided with apertures **13** which are aligned with the dispensing apertures **2** of the lid **1**. These foils **12,** when assembling the entire holder with the membrane **11** for the transfer of biomolecules, constitute the bottom wall of the discharge channels **4** and/or of the collecting channels **5** and/or of the sealing chambers **6,** and at the same time prevent soaking the liquid being sucked out ino the membrane **11** for the transfer of biomolecules outside the dispensing apertures **2** of the lid **1** and at the same time prevent deformation of the dots formed thereon.

Under the base **8** of the holder of the membrane **11** for the transfer of biomolecules is mounted a known bottom **14** of the holder **1** which is on its upper side provided with an unillustrated depression which is arranged under the through apertures **9** in the base **8.** This depression is connected to at least one discharge aperture **15** of the bottom **14** for draining the liquid that has passed through the holder of the membrane **11** for the transfer of biomolecules, by means of a vacuum source (not shown) with an interposed safety vessel. The space between the base **8** and the bottom **14** of the holder is sealed with a suitable seal, preferably made of microporous rubber.

The base **8** and the bottom **14** of the holder of the membrane **11 for** the transfer of biomolecules are preferably connected detachably (for washing) - in the embodiment shown in Fig. 2, for example by means of four screws. In addition, in the base **8** are mounted threaded pins or screws **16** or other means which project above the upper surface of the base 8 and allow removable connection of the lid **1** provided with the corresponding connecting apertures **160,** preferably, but not necessarily, e.g. by means of wing nuts, which allow the operator to manually connect and detach the lid **1**. The threaded pins or screws **16,** or other means of the base **8** and the connecting apertures **160** of the lid **1** are arranged in a square matrix, so that the lid **1** can be connected to the base **8** ,e.g., in cases when a chaotropic step is included in the analysis even after the lid has been rotated by 90 ° in a horizontal plane.

To use the holder of the membrane **11** for the transfer of biomolecules according to the invention, the individual parts of the holder are first assembled, whereby the membrane **11** for the transfer of biomolecules is inserted between the lid **1** and the base **8** - if the discharge channels **4** and/or the collecting channels **5** and/or the sealing chambers **6** are formed on the underside of the lid **1** as open, the membrane **11** for the transfer of biomolecules is inserted between two layers **12** of foil with apertures **13** aligned with the dispensing apertures **2** of the lid **1,** or a pre-prepared membrane **11** for the transfer of biomolecules which has been provided in advance with such layers **12** of foil is used, and the discharge aperture/apertures **3** of the lid **1** and discharge aperture/apertures **15** of the bottom **14** are connected to the vacuum source.

In performing the dot blot technique, to determine the degree of mutual reactivity of the target and complementary biomolecules, first a solution of a target biomolecule (e.g., protein, peptide, sugar, nucleic acid and the like) is pipetted through the dispensing apertures **2** onto the membrane **11** for the transfer of biomolecules, which is fixed in the holder according to the invention. Thereafter, the bottom **14** of the holder is connected to the vacuum source, and consequently the biomolecule solution is filtered through the membrane **11** for the transfer of biomolecules to which the biomolecules are fixed. Subsequently, a suitable blocking reagent (e.g., a 1% albumin solution) is applied to the biomolecule containing dots on the membrane **11** for the transfer of biomolecules through the dispensing containers **7** on the upper side of the lid **1** of the holder of the membrane **11** and/or through the dispensing apertures **2.** The blocking reagent is after the appropriate incubation time drained off the surface of the membrane 11 for the transfer of biomolecules through the discharge channels 4 and the discharge aperture/apertures 3 of the lid 1 into a vessel (not shown) by the action of the vacuum source. Afterwards, the complementary biomolecules to be tested are applied to these biomolecule containing dots in a suitable manner by means of the dispensing containers 7 on the upper side of the lid 1 of the holder of the membrane 11 for the transfer of biomolecules and/or by means of the dispensing apertures 2, whereby the complementary biomolecules being applied have the ability to bind only a particular target molecule or a group of target molecules (wherein each complementary molecule is applied to the dispensing apertures 2 in a single line), which after the respective incubation time are drained off the surface of the membrane **11** for the transfer of biomolecules through the discharge channels **4** and the discharge aperture/apertures **3** of the lid into a vessel (not shown) connected via the vacuum source. Subsequently, the biomolecule containing dots on the membrane **11** for the transfer of biomolecules are washed with a washing solution and the binding resistance of the target and complementary biomolecules (e.g., a specific protein and complementary antibody) is tested, rotating the lid **2** of the holder of the membrane **11** for the transfer of biomolecules by 90 °. As a result, the direction of application of the solutions from the dispensers **7** is changed from horizontal to vertical (or vice versa), and a different concentration of chaotropic reagent is introduced into these dispensing containers **7.** After a short period of incubation (in the order of minutes) the chaotropic reagent is sucked out from the surface of the membrane **11** for the transfer of biomolecules through the discharge channels **4** and the discharge aperture/apertures **3** of the lid **1** into a vessel (not shown) connected via the vacuum source. Subsequently, a suitable detection reagent of the target and complementary biomolecule complex (e.g., an enzyme-labelled secondary antibody) is then introduced into the dispensing apertures **2** and after another incubation, further washing with the washing solution follows. Finally, the membrane **11** for the transfer of biomolecules is removed from the holder and is immersed in a solution containing a substrate and chromogen which, depending on the amount of the target and complementary biomolecule complexes, forms a colored dot on the membrane **11** for the transfer of biomolecules. The color intensity of this dot corresponds to the concentration of the complex and by means of a densitometric analysis it is possible to determine from it the degree of resistance of the binding in relation to the concentration of the chaotropic reagent. Complementary molecules whose dots remain dark even after incubation with a higher concentration of chaotropic reagent may be considered suitable for use in further assays. It is then appropriate to verify the possibility of binding the complementary biomolecule to a non-target biomolecule (the so-called selectivity or cross reactivity), i.e., the ability to bind only to a target biomolecule - not to other biomolecules potentially present in the sample together with the target molecule.

In performing the dot blot technique to verify the possibilities of the binding of a complementary biomolecule with a non-target biomolecule, solutions of different biomolecules which are potentially present in the sample with the target biomolecule but which should not bind to the complementary molecule are applied to each line through the dispensing apertures **2** onto the membrane **11** for the transfer of biomolecules; the target biomolecule, which serves as a positive control, is applied through one dispensing aperture **2** onto the membrane **11** for the transfer of biomolecules. Thereafter, the above-described process is performed, without a step with the chaotropic reagent. The complementary biomolecule should only react with the target biomolecule (which results in a strong coloring of the dot); dots with other biomolecules should not be colored. If other dots get colored, cross reactivity can be expected in this complementary biomolecule. It is then possible to select biomolecules suitable for specific scientific work from comparing the results obtained by comparing the degree of mutual reactivity and verifying the possibility of binding to a non-target biomolecule.

Another possible use of the holder of the membrane **11** for the transfer of biomolecules according to the invention is a classic technique of dot blot, in which samples of one's choice are applied through the dispensing apertures **2** to the membrane **11** for the transfer of biomolecules and then analyzed by one or more complementary molecules of one's choice. The membrane **11** for the transfer of biomolecules stays in the holder all the time and the washing and replacement of the solutions takes place by sucking them through the discharge channels **4** and the discharge aperture/apertures **3** of the lid **1.** The step with a chaotropic reagent is omitted here.

The lid **1** of the holder of the membrane **11** for the transfer of biomolecules and the holder of the membrane **11** for the transfer of biomolecules according to the invention allow, in addition to the classical technique of dot blot, wherein the dots of the samples are washed and made visible with a labelling reagent, to perform also more complicated processes including, for example, application of titration series of reagents to dispensed samples, or application of samples to affinity targets prepared by vacuum-assisted extraction through the membrane **11** for the transfer of biomolecules and many other methods.

### List of references

- 1: lid of a holder of a membrane for the transfer of biomolecules
- 10: recess of the lid of the holder of the membrane for the transfer of biomolecules
- 2: dispensing aperture
- 3: discharge aperture
- 30: quick coupling
- 4: discharge channel
- 5: collecting channel
- 6: sealing chamber
- 7: dispensing container
- 8: base of the holder of the membrane for the transfer of biomolecules
- 80: elevation of the base of the holder of the membrane for the transfer of biomolecules
- 9: through aperture
- 11: membrane for the transfer of biomolecules
- 12: foil
- 13: aperture in the foil
- 14: bottom of the holder of the membrane for the transfer of biomolecules
- 15: discharge aperture of the bottom of the holder of the membrane for the transfer of biomolecules
- 16: threaded pin or screw
- 160: connecting aperture

## Claims

1. A lid (1) of a holder of a membrane (11) for performing a technique of dot blot, in which through dispensing apertures (2) and at least one discharge aperture (3) are formed, **characterized in that** the through dispensing apertures (2) are connected to the discharge aperture (3) in the vicinity of their orifice on the underside of the lid (1) either directly via discharge channels (4) or via discharge channels (4) and collecting channels (5), whereby discharge channels (4) are connected to collecting channels (5), which are connected to the discharge aperture (3), whereby collecting channels (5) have a larger cross-sectional area than discharge channels (4), whereby the discharge channels (4) are made as open channels on the underside of the lid (1) or configured as closed channels in the body of the lid (1), whereby each discharge channel (4) has a cross-sectional area having a size of a maximum of 12000 µm² and is provided with at least one sealing chamber (6) and/or with a hydrophobic surface coating or finish on at least a portion of its length, the sealing chamber (6) and/or hydrophobic surface coating or finish preventing the backflow to the dispensing aperture (2), whereby if the discharge channels (4) and/or the collecting channels (5) and/or sealing chambers (6) of the discharge channels (4) are made as open, the lid (1) is designed for use in combination of a membrane (11) for the transfer of biomolecules arranged between two layers of foil (12) which is provided with apertures (13) which are aligned with the dispensing apertures (2) of the lid (1).

2. The lid (1) of the holder of the membrane (11) according to claim 1, **characterized in that** the dispensing apertures (2) open into a recess formed on the underside of the lid (1).

3. The lid (1) of the holder of the membrane (11) according to claim 1 or 2, **characterized in that** the dispensing apertures (2) are arranged in the lid (1) in a square matrix.

4. The lid (1) of the holder of the membrane (11) according to claim 1, **characterized in that** each discharge channel (4) has a cross-sectional area having a size of a maximum of 10000 µm².

5. The lid (1) of the holder of the membrane (11) according to claim 1 or 4, **characterized in that** each discharge channel (4) has a rectangular cross-section with a width of 200 µm and a depth of 50 µm.

6. The lid (1) of the holder of the membrane (11) according to claim 1, **characterized in that** the discharge aperture/apertures (3) is/are provided with a quick coupling (30).

7. The lid (1) of the holder of the membrane (11) according to claim 1 or 6, **characterized in that** the discharge aperture/apertures (3) is/are formed in at least one lateral side of the lid (1).

8. The lid (1) of the holder of the membrane (11) according to claim 1, **characterized in that** a dispensing container (7) is formed for each dispensing aperture (2) in the upper side of the lid (1).

9. The lid (1) of the holder of the membrane (11) according to claim 8, **characterized in that** at least some dispensing containers (7) are common to several dispensing apertures (2).

10. A membrane holder for performing a technique of dot blot, which comprises a lid (1) with through dispensing apertures (2), a base (8) with through apertures (9) which are aligned with the dispensing apertures (2) of the lid (1) and a bottom (14) with a recess arranged under the through apertures (9) of the base (8), which is connected to a discharge aperture (15), **characterized in that** the lid (1) of the holder is formed by the lid (1) according to any of claims 1 to 9.

11. The membrane holder for performing the technique of dot blot according to claim 10
**characterized in that** the discharge channels (4) are made as open channels on the underside of the lid (1), whereby a membrane (11) for the transfer of biomolecules is arranged between the lid (1) and the base (8) of the holder, the membrane (11) being arranged between two layers of foil (12) which are provided with apertures (13) which are aligned with the dispensing apertures (2) of the lid (1).

## Patentansprüche

1. Deckel (1) eines Halters einer Membrane (11) zur Durchführung des Verfahrens zur Absaugung von Flecken, in dem durchgehende Dosieröffnungen (2) und mindestens eine Abfallöffnung (3) gebildet sind, **dadurch gekennzeichnet, dass** die durchgehenden Dosieröffnungen (2) mit dieser Abfallöffnung (3) in der Nähe von ihrer Mündung auf der unteren Seite des Deckels (1) verbunden sind, und zwar entweder direkt mittels der Abfallkanäle (4) oder mittels der Abfallkanäle (4) und der Sammelkanäle (5), wobei die Abfallkanäle (4) mit den Sammelkanälen (5) verbunden sind, die mit der Abfallöffnung (3) verbunden sind, wobei die Sammelkanäle (5) eine größere Querschnittsfläche als die Abfallkanäle (4) aufweisen, wobei die Abfallkanäle (4) als geöffnete Kanäle auf der unteren Seite des Deckels (1) oder als geschlossene Kanäle im Körper des Deckels (1) gebildet sind, wobei jeder Abfallkanal (4) eine Querschnittsfläche mit einer Größe von maximal 12000 µm² und mindestens eine Dichtungskammer (6) und/oder mindestens auf einem Teil von seiner Länge eine hydrophobe Oberflächenschicht oder eine Aufbereitung aufweist, wobei Dichtungskammer (6) und/oder hydrophobe Oberflächenschicht oder Aufbereitung an einem Rückfluss in die Dosieröffnung (2) hindern, wobei wenn die Abfallkanäle (4) und/oder die Sammelkanäle (5) und/oder die Dichtungskammern (6) der Abfallkanäle (4) als geöffnet gebildet sind, ist der Deckel (1) zur Verwendung in der Kombination mit einer Membrane (11) zur Übertragung von Biomolekülen vorgesehen, die zwischen zwei Schichten einer Folie (12) gelagert ist, die die Öffnungen (13) aufweist, die mit den Dosieröffnungen (2) des Deckels (1) fluchten.

2. Deckel (1) eines Halters einer Membrane (11) nach dem Anspruch 1, **dadurch gekennzeichnet, dass** die Dosieröffnungen (2) in einer Ausnehmung ausgemündet sind, die auf der unteren Seite des Deckels (1) gebildet ist.

3. Deckel (1) eines Halters einer Membrane nach dem Anspruch 1 oder 2, **dadurch gekennzeichnet, dass** die Dosieröffnungen (2) in dem Deckel (1) in einer Quadratmatrix angeordnet sind.

4. Deckel (1) eines Halters einer Membrane (11) nach dem Anspruch 1, **dadurch gekennzeichnet, dass** jeder Abfallkanal (4) eine Querschnittsfläche mit einer Größe von höchstens 10000 µm² aufweist.

5. Deckel (1) eines Halters einer Membrane (11) nach dem Anspruch 1 oder 4, **dadurch gekennzeichnet, dass** jeder Abfallkanal (4) einen Rechteckquerschnitt mit einer Breite von 200 µm und Tiefe von 50 µm aufweist.

6. Deckel (1) eines Halters einer Membrane (11) nach dem Anspruch 1, **dadurch gekennzeichnet, dass** die Abfallöffnung/-öffnungen (3) eine Schnellkupplung (30) aufweist/aufweisen.

7. Deckel (1) eines Halters einer Membrane (11) nach dem Anspruch 1 oder 6, **dadurch gekennzeichnet, dass** die Abfallöffnung/-öffnungen (3) in mindestens einer Seitenwand des Deckels (1) gebildet ist/sind.

8. Deckel (1) eines Halters einer Membrane (11) nach dem Anspruch 1, **dadurch gekennzeichnet, dass** in der oberen Seite des Deckels (1) für jede Dosieröffnung (2) ein Dosierbehälter (7) gebildet ist.

9. Deckel (1) eines Halters einer Membrane (11) nach dem Anspruch 8, **dadurch gekennzeichnet, dass** mindestens einige Dosierbehälter (7) für mehrere Dosieröffnungen (2) gemeinsam sind.

10. Halter einer Membrane zur Durchführung des Verfahrens zur Absaugung von Flecken, der Deckel (1) mit durchgehenden Dosieröffnungen (2), Fundament (8) mit durchgehenden Öffnungen (9), die mit Dosieröffnungen (2) des Deckels (1) fluchten, und Boden (14) mit einer Ausnehmung aufweist, die unter den durchgehenden Öffnungen (9) des Fundaments (8) angeordnet ist, wobei die Ausnehmung mit einer Abfallöffnung (15) verbunden ist, **dadurch gekennzeichnet, dass** der Deckel (1) von diesem Halter durch den Deckel (1) nach einem der Ansprüche 1 bis 9 gebildet wird.

11. Halter einer Membrane zur Durchführung des Verfahrens zur Absaugung von Flecken nach dem Anspruch 10, **dadurch gekennzeichnet, dass** die Abfallkanäle (4) als geöffnete Kanäle auf der unteren Seite des Deckels (1) gebildet sind, wobei zwischen dem Deckel (1) und dem Fundament (8) von diesem Halter eine Membrane (11) zur Übertragung von Biomolekülen gelagert ist, die zwischen zwei Schichten der Folie (12) gelagert ist, die die Öffnungen (13) aufweisen, die mit den Dosieröffnungen (2) des Deckels (1) fluchten.

## Revendications

1. Couvercle (1) d'un support de membrane (11) pour la mise en place du procédé d'aspiration de taches, dans lequel sont formées des ouvertures traversantes de distribution (2) et au moins une ouverture d'évacuation (3), **caractérisé en ce que** les ouvertures traversantes de distribution (2) sont reliées à l'ouverture d'évacuation (3) près de leur embouchure sur la face inférieure du couvercle (1), soit directement par des canaux d'évacuation (4), soit par des canaux d'évacuation (4) et des canaux collecteurs (5), où les canaux d'évacuation (4) sont reliés aux canaux collecteurs (5) qui sont reliés à l'ouverture d'évacuation (3), où les canaux collecteurs (5) disposent d'une surface de section transversale plus grande que les canaux d'évacuation (4), où les canaux d'évacuation (4) sont formés comme des canaux ouverts sur la face inférieure du couvercle (1) ou comme des canaux fermés dans le corps du couvercle (1), où chaque canal d'évacuation (4) dispose d'une section transversale de 12000 µm² au maximum et est muni d'au moins une chambre d'étanchéité (6) et/ou d'un revêtement ou traitement hydrophobe sur au moins une partie de sa longueur, où la chambre d'étanchéité (6) et/ou le revêtement ou traitement hydrophobe empêchent le reflux dans l'ouverture de distribution (2), et lorsque les canaux d'évacuation (4) et/ou les canaux collecteurs (5) et/ou les chambres d'étanchéité (6) des canaux d'évacuation (4) sont formés comme étant ouverts, le couvercle (1) doit être utilisé en combinaison avec une membrane (11) pour le transfert de biomolécules, disposée entre deux couches d'un film (12) avec ouvertures (13) qui font face aux ouvertures de distribution (2) du couvercle (1).

2. Couvercle (1) d'un support de membrane (11) selon la revendication 1, **caractérisé en ce que** les débouchés des ouvertures de distribution (2) se situent dans un évidement formé sur la face inférieure du couvercle (1).

3. Couvercle (1) d'un support de membrane selon la revendication 1 ou 2, **caractérisé en ce que** les ouvertures de distribution (2) sont disposées dans une matrice carrée dans le couvercle (1).

4. Couvercle (1) d'un support de membrane (11) selon la revendication 1, **caractérisé en ce que** chaque canal d'évacuation (4) dispose d'une surface de section transversale de 10000 µm² au maximum.

5. Couvercle (1) d'un support de membrane (11) selon la revendication 1 ou 4, **caractérisé en ce que** chaque canal d'évacuation (4) dispose d'une section transversale rectangulaire avec une largeur de 200 µm et une profondeur de 50 µm.

6. Couvercle (1) d'un support de membrane (11) selon la revendication 1, **caractérisé en ce que** la ou les ouvertures d'évacuation (3) est/sont munie/s d'un raccord rapide (30).

7. Couvercle (1) d'un support de membrane (11) selon la revendication 1 ou 6, **caractérisé en ce que** la ou les ouvertures d'évacuation (3) est/sont formée/s dans au moins une paroi latérale du couvercle (1).

8. Couvercle (1) d'un support de membrane (11) selon la revendication 1, **caractérisé en ce qu'**un réservoir de distribution (7) est formé dans la face supérieure du couvercle (1) pour chaque ouverture de distribution (2).

9. Couvercle (1) d'un support de membrane (11) selon la revendication 8, **caractérisé en ce qu'**au moins certains des réservoirs de distribution (7) sont communs pour plusieurs ouvertures de distribution (2).

10. Support de membrane pour la mise en place du procédé d'aspiration de taches comprenant un couvercle (1) avec des ouvertures traversantes de distribution (2), une base (8) avec des trous traversants (9) qui font face aux ouvertures de distribution (2) du couvercle (1), et un fond (14) avec un évidement disposé sous les ouvertures traversantes (9) de la base (8), où l'évidement est relié à l'ouverture d'évacuation (15), **caractérisé en ce que** le couvercle (1) de ce support est formé par un couvercle (1) selon l'une des revendications de 1 à 9.

11. Support de membrane pour la mise en place du procédé d'aspiration de taches selon la revendication 10, **caractérisé en ce que** les canaux d'évacuation (4) sont formés comme des canaux ouverts sur la face inférieure du couvercle ( 1), où, entre le couvercle (1) et la base (8) de ce support est prévue une membrane (11) pour le transfert de biomolécules, qui est disposée entre deux couches d'un film (12) avec ouvertures (13) qui font face aux ouvertures de distribution (2) du couvercle (1).
